# EUROPEAN PATENT APPLICATION

(11) **EP 0 848 001 A1**
(43) Date of publication of application: **17.06.1998**
(21) Application number: 97118048.4
(22) Date of filing: 17.10.1997
(51) Int. Cl.: C07D 405/12

(54) **Crystalline form of the doxazosin mesylate and process for its production**

(30) Priority: 13.12.1996 IT BO960657
(71) Applicant: Alfa Chemicals Italiana S.r.l., 24122 Bergamo (IT)
(72) Inventor: Cannata, Vincenzo, Pontecchio Marconi, Bologna (IT); Piani, Silvano, 40131 Bologna (IT); Carrara, Cristina, 40012 Calderara di Reno (Bologna) (IT); Saguatti, Stefano, 40033 Casalecchio di Reno (Bologna) (IT)
(74) Representative: Kraus, Walter, Dr.

(57) **Abstract**

The invention refers to a crystalline form of the doxazosin mesylate and to a process for its production by hot treating with methanesulfonic acid doxazosin suspended in a solvent selected from the alcohols, straight or branched, from C₁ to C₆, the esters of the aliphatic carboxylic acids from C₁ to C₈ with the alcohols, straight or branched, from C₁ to C₈, the aliphatic ketones from C₃ to C₈ and mixtures thereof.

## Description

### BACKGROUND OF THE INVENTION

The compound 1-(4-amino-6,7-dimethoxy-2-quinazolinyl)-4-[(2,3-dihydro-1,4-benzodioxin-2-yl)carbonyl]piperazine is internationally known under the name of doxazosin. This compound, together with its pharmaceutically acceptable salts, has been described in US Patent 4,188,390. The product has been studied as antihypertensive drug and has been subsequently marketed everywhere in the world under many trademarks, in Italy and the United States under the trademark CARDURA®, under form salified with the methanesulfonic acid named doxazosin mesylate, whose synthesis and chemical-physical characteristics result to be never made known.

### DESCRIPTION OF THE INVENTION

A crystalline form of the 1-(4-amino-6,7-dimethoxy-2-quinazolinyl)-4-[(2,3-dihydro-1,4-benzodioxin-2-yl)carbonyl]piperazine monomethanesulfonate, named doxazosin mesylate, and a process for its production are the object of the present invention.

This crystalline form has been characterized through three kinds of structural analytical techniques: powder X-ray diffraction, IR spectrum and differential thermal analysis.

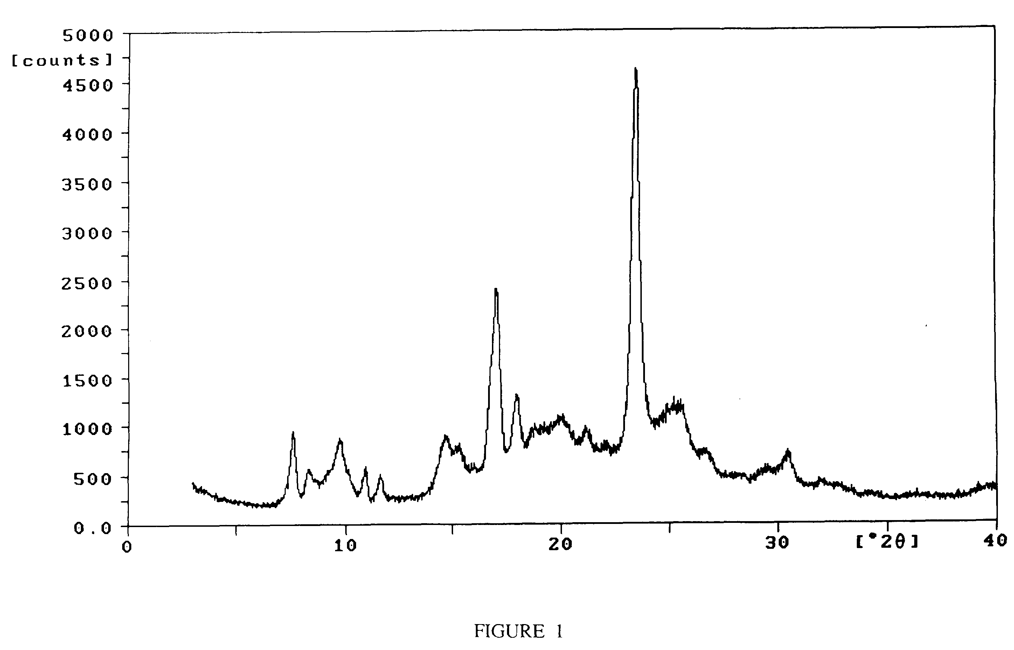

The technique of powder X-ray diffraction has been carried out by means of an automatic powder diffractometer Philips model PW1050, controlled by a PW1710 unit, with Bragg-Brentano geometry, by means of monochromatic CuK_{α} radiation (wavelenght 1.54060 Å, 40 kV and 40 mA) with scansion interval 3-40 in 2 θ in degrees, angular pitch 0.02 degrees and scansion time 1.25 seconds for angular pitch and at room temperature. The samples have been dry prepared by light grinding in mortar of agate without compression. In the diffractogram of figure 1 the intensities of the diffracted X-rays (counts) are reported in function of the diffraction angle 2 θ.

The more significant diffraction angles 2 θ, with an approximation of ± 0.2°, obtained in the powder X-ray diffraction, are as follows:
7.67°; 8.30°; 9.76°; 10.91°; 11.61°; 14.70°; 15.28°; 17.05°; 17.93°; 23.51° and 30.48°.

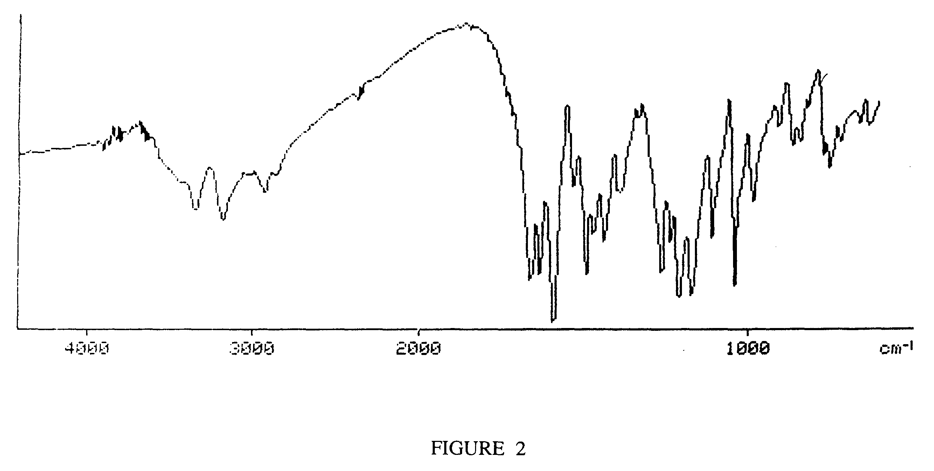

The IR spectrum, reported in figure 2, has been carried out with a FT-IR Perkin Elmer 6100 spectrophotometer on samples containing 0.3% of product in KBr and with registration between 4400 and 600 cm⁻¹.

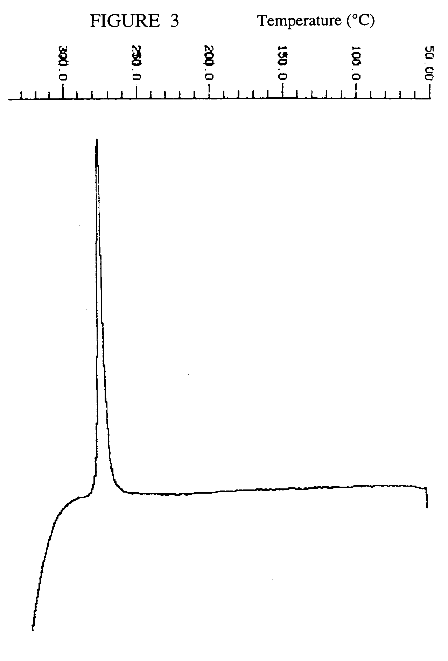

The differential thermal analysis, with the thermogram reported in figure 3, has been carried out with an instrument DSC METTLER TA 4000, starting from an initial temperature of 50°C until the temperature of 340°C and with a scansion speed equal to 5°C/min. The analysis has been carried out on a holed melting-pot containing an amount of substance between 4 and 6 mg. The product shows a melting temperature equal to 270°C±1°C.

The process for the production of the above defined crystalline form starts by suspending the 1-(4-amino-6,7-dimethoxy-2-quinazolinyl)-4-[(2,3-dihydro-1,4-benzodioxin-2-yl) carbonyl]piperazine in a solvent selected from the alcohols, straight or branched, from C₁ to C₆, the esters of the aliphatic carboxylic acids from C₁ to C₈ with the alcohols, straight or branched, from C₁ to C₈, the aliphatic ketones from C₃ to C₈ and mixtures thereof and by heating the resulting suspension at a temperature between 50°C and the boiling temperature under stirring and optionally under nitrogen atmosphere.

In a preferred aspect of the invention the solvent is selected from methanol, ethanol, isopropanol, n-butanol, n-butyl acetate, acetone, methylisobutylketone or mixtures thereof and the amount by volume of solvent is between 5 and 15 times in respect of the weight of the 1-(4-amino-6,7-dimethoxy-2-quinazolinyl)-4-[(2,3-dihydro-1,4-benzodioxin-2-yl)carbonyl]piperazine.

The suspension, under stirring and optionally under nitrogen atmosphere, is added with from 0.95 to 1.05 equivalents of methanesulfonic acid in respect of the 1-(4-amino-6,7-dimethoxy-2-quinazolinyl)-4-[(2,3-dihydro-1,4-benzodioxin-2-yl)carbonyl]piperazine, while keeping the temperature between 50°C and the boiling temperature.

The reaction mixture is further heated at the end of the addition at a temperature between 50°C and the boiling temperature for a period of time between 10 minutes and 3 hours and, optionally, the solvent is partially distilled off.

The suspension is then cooled at a temperature between 0°C and 20°C under stirring, optionally under nitrogen atmosphere, and filtered. The crystalline solid is washed with the solvent and dried in oven under vacuum at a temperature between 50°C and 75°C for a period of time between 12 and 24 hours.

The examples underneath reported have to be considered as a further illustration of the invention and not as an its limitation.

### EXAMPLE 1

40 Grams of 1-(4-amino-6,7-dimethoxy-2-quinazolinyl)-4-[(2,3-dihydro-1,4-benzodioxin-2-yl)carbonyl]piperazine are suspended, under nitrogen atmosphere, in 400 ml of ethanol in a reactor equipped with steam-jacket, stirrer and cooling column and the suspension is heated to 55°C under stirring and then is added with 8.8 g of methanesulfonic acid. The reaction mixture is kept at 60°C for one hour, then it is cooled to 5°C under stirring and filtered. The solid is washed with 90 ml of ethanol and dried for 24 in oven under vacuum at the temperature of 50°C.

46 Grams of crystals of 1-(4-amino-6,7-dimethoxy-2-quinazolinyl)-4-[(2,3-dihydro-1,4-benzodioxin-2-yl)carbonyl]piperazine monomethanesulfonate are obtained with a yield equal to 94.8%. The chemical-physical characteristics of the product obtained are reported in figures 1, 2 and 3.

### EXAMPLE 2

150 Grams of 1-(4-amino-6,7-dimethoxy-2-quinazolinyl)-4-[(2,3-dihydro-1,4-benzodioxin-2-yl)carbonyl]piperazine are suspended in 1500 ml of n-butanol in the reactor described in example 1 and the mixture is heated until 90°C under stirring and then is added with 33.5 g of methanesulfonic acid. The reaction mixture is kept at 90°C for 15 minutes, then cooled to 5°C under stirring and filtered. The solid is washed with 200 ml of n-butanol and dried for 24 hours in oven under vacuum at the temperature of 55°C. 171.6 Grams of crystals of 1-(4-amino-6,7-dimethoxy-2-quinazolinyl)-4-[(2,3-dihydro-1,4-benzodioxin-2-yl)carbonyl]piperazine monomethanesulfonate are obtained with a yield equal to 94.3%. The product shows the same chemical-physical characteristics as those of the product described in example 1.

### EXAMPLE 3

11.64 Grams of 1-(4-amino-6,7-dimethoxy-2-quinazolinyl)-4-[(2,3-dihydro-1,4-benzodioxin-2-yl)carbonyl]piperazine are suspended in a mixture made by 40 ml of methanol and 75 ml of n-butyl acetate. The suspension is heated to the boiling temperature and in the period of 30 minutes 23.86 ml of a 10% (w/v) methanolic solution of methanesulfonic acid are added and the mixture is kept at the boiling temperature for another hour. Then the solvent mixture is partially distilled off until a temperature of distillation equal to 95°C is reached. The suspension is then cooled to 20°C under nitrogen atmosphere and filtered. The crystalline solid is washed with 15 ml of n-butyl acetate and dried in oven under vacuum at 70°C for 12 hours obtaining 12.2 g of product having the same chemical-physical characteristics as those of the product described in example 1 with a yield equal to 86.4%.

### EXAMPLE 4

The experiment of example 3 is repeated by substituting the n-butyl acetate with the following solvents: acetone, yield equal to 88.9%; isopropanol, yield equal to 94.6%; n-butanol, yield equal to 95.4%; methylisobutylketone, yield equal to 97.9%.

All the products obtained show the same chemical-physical characteristics as those of the product described in example 1.

## Claims

1. Crystalline form of the 1-(4-amino-6,7-dimethoxy-2-quinazolinyl)-4-[(2,3-dihydro-1,4-benzodioxin-2-yl)carbonyl]piperazine monomethanesulfonate characterized by diffraction angles 2 θ, in the powder X-ray diffraction, having the following values: 7.67°±0.2°; 8.30°±0.2°; 9.76°±0.2°; 10.91°±0.2°; 11.61°±0.2°; 14.70°±0.2°; 15.28°±0.2°; 17.05°±0.2°; 17.93°±0.2°; 23.51°±0.2° and 30.48°±0.2° and from a melting point equal to 270°C±1°C.

2. Process for the production of the crystalline form of the 1-(4-amino-6,7-dimethoxy-2-quinazolinyl)-4-[(2,3-dihydro-1,4-benzodioxin-2-yl)carbonyl] piperazine monomethanesulfonate as defined in claim 1, which comprises:
(a) heating, optionally under nitrogen atmosphere, at a temperature between 50°C and the boiling temperature a suspension of 1-(4-amino-6,7-dimethoxy-2-quinazolinyl)-4-[(2,3-dihydro-1,4-benzodioxin-2-yl)carbonyl]piperazine in a solvent selected from the alcohols, straight or branched, from C₁ to C₆, the esters of the aliphatic carboxylic acids from C₁ to C₈ with the alcohols, straight or branched, from C₁ to C₈, the aliphatic ketones from C₃ to C₈ and mixtures thereof and adding under stirring from 0.5 to 1.05 equivalents of methanesulfonic acid, while keeping the temperature between 50°C and the boiling temperature; (b) going on with the heating at a temperature between 50°C and the boiling temperature for a period of time between 10 minutes and 3 hours, optionally distilling off the solvent partially; (c) cooling, optionally under nitrogen atmosphere, at a temperature between 0°C and 20°C the suspension under stirring, filtering it, washing the solid with the solvent and drying it in oven under vacuum at a temperature between 50°C and 75°C for a period of time between 12 and 24 hours.

3. Process according to claim 2 characterized in that the solvent is selected from methanol, ethanol, isopropanol, n-butanol- n-butyl acetate, acetone, methylisobutylketone or mixtures thereof and that the amount by volume of solvent is between 5 and 15 times with respect to the weight of the 1-(4-amino-6,7-dimethoxy-2-quinazolinyl)-4-[(2,3-dihydro-1,4-benzodioxin-2-yl)carbonyl] piperazine.
